# EUROPEAN PATENT APPLICATION

(11) **EP 2 684 951 A1**
(43) Date of publication of application: **15.01.2014**
(21) Application number: 12176360.1
(22) Date of filing: 13.07.2012
(51) Int. Cl.: C12N 9/50, C12N 15/62, C12P 21/06

(54) **Method for producing a recombinant protein of interest**

(71) Applicant: SANDOZ AG, 4056 Basel (CH)
(72) Inventor: Keller, Sascha, 6020 Innsbruck (AT); Funke, René, 83080 Oberaudorf (DE)
(74) Representative: Sonn & Partner Patentanwälte

(57) **Abstract**

Disclosed is a method for producing a recombinant protein of interest, characterised in by the following steps:
(a) providing a fusion protein comprising an N^{pro} autoprotease moiety and a protein of interest moiety in inclusion bodies,
(b) solubilising the fusion protein in the inclusion bodies by subjecting the inclusion bodies to chaotropic conditions,
(c) binding the fusion protein of the solubilised inclusion bodies to a multimodal chromatographic material under chaotropic conditions,
(d) eluting the fusion protein from the multimodal chromatographic material with an elution buffer and allowing the fusion protein to be cleaved by the N^{pro} autoprotease moiety under kosmotropic conditions, wherein the recombinant protein of interest is cleaved from the fusion protein, and
(e) recovering the protein of interest.

## Description

The present invention relates to a process for the recombinant production of a desired heterologous polypeptide of interest by using the autoprotease N^{pro} of Pestivirus-technology.

Overexpression of heterologous proteins in E. coli frequently leads aggregation and deposition in dense, insoluble particles, also known as inclusion bodies. Advantages of the expression in inclusion bodies are the high purity of the desired product and the easy purification by centrifugation after cell disruption. However, crucial steps are resolving and refolding of the protein into its native structure. Solubilisation usually is carried out in high concentrations of chaotropic agents like urea or guanidinium hydrochloride to reach complete unfolding. Reducing agents such as 2-mercaptoethanol (β-ME), dithiothreitol (DTT) or 1-monothioglycerol (MTG) are added to reduce non-native inter- and intramolecular disulfide bonds and keep the cysteins in a reduced state.

A bottleneck step is the renaturation of the proteins. Elimination of hydrophobic intermolecular interaction during the first steps of refolding is crucial for successful renaturation at high protein concentrations and to prevent aggregation (Vallejo et al., Microb. Cell Fact. 3 (2004), 11). Several renaturation techniques are known.

A technology platform was established by using the genetically engineered Npro autoprotease from classical swine fever virus (CSFV) to produce difficult-to-express therapeutic peptides and proteins in form of inclusion bodies in E. coli. This fusion protein technology processing requires renaturation of the inclusion bodies, autoprotease cleavage and refolding of the released target molecule which is usually performed in batch mode. Due to its simplicity refolding by dilution is preferred to pressure treatment or chromatographic techniques, especially in production scale. Protein concentration, as well as chaotrop concentration are diminished in a single step preventing aggregation by intermolecular interactions. However, large volumes and low protein concentration burden downstream processing steps. (Jungbauer et al., J. Biotech. 128 (2007), 587-596).

It is therefore an object of the present invention to provide an improvement in renaturation of inclusion bodies which must be renaturated, especially for autoproteolytic cleavage and preparation of recombinant proteins downstream of the process. Preferably, the invention should enable low volumes and high protein concentrations for obtaining the protein of interest and provide a method which is suitable to be established in industrial production scale, specifically for proteins used in medicine.

Therefore, the present invention provides a method for producing a recombinant protein of interest, characterised in by the following steps:
(a) providing a fusion protein comprising an N^{pro} autoprotease moiety and a protein of interest moiety in inclusion bodies,
(b) solubilising the fusion protein in the inclusion bodies by subjecting the inclusion bodies to chaotropic conditions,
(c) binding the fusion protein of the solubilised inclusion bodies to a multimodal chromatographic material under chaotropic conditions,
(d) eluting the fusion protein from the multimodal chromatographic material with an elution buffer and allowing the fusion protein to be cleaved by the N^{pro} autoprotease moiety under kosmotropic conditions, wherein the recombinant protein of interest is cleaved from the fusion protein, and
(e) recovering the protein of interest.

The present invention is an improvement in the recombinant production of a desired heterologous polypeptide of interest by using the autoprotease N^{pro} of Pestivirus-technology. This technology usually provides the recombinant expression of a fusion polypeptide which comprises an autoproteolytic moiety directly or indirectly derived from autoprotease N^{pro} of Pestivirus and a heterologous polypeptide of interest in a host cell, often a prokaryotic host cell, such as E. coli. The heterologous polypeptide or protein of interest is covalently coupled via a peptide bond to the N^{pro} molecule. The protein of interest is released from the fusion protein through hydrolysis of the peptide bond between the C-terminal Cys168 of N^{pro} and position 169 of the fusion polypeptide which represents the authentic N-terminal amino acid of the protein of interest to be produced according to the present invention. The heterologous polypeptide of interest is produced in the host cell in form of cytoplasmic inclusion bodies (IB), which are then isolated and treated in such a way, that the desired heterologous polypeptide is cleaved from the fusion polypeptide by the N^{pro} autoproteolytic activity.

Fusion polypeptides comprising the autoprotease Npro of Pestivirus are therefore specifically useful for producing heterologous recombinant polypeptides. N^{pro} is an autoprotease with length of 168 amino acids and an apparent Mᵣ of about 20 kD in vivo. It is the first protein in the polyprotein of Pestiviruses and undergoes autoproteolytic cleavage from the following nucleocapsid protein C. This cleavage takes place after the last amino acid in the sequence of N^{pro}, Cys168. The autoprotease N^{pro} activity of Pestivirus always cleaves off the fusion partner at this clearly determined site, releasing a polypeptide of interest with homogenous N-terminus. In addition, the autoproteolytic activity of N^{pro} can be induced in vitro, by application of special buffers, so that the polypeptide of interest can be obtained by cleavage of fusion polypeptides that are expressed in IBs.

N-terminal autoprotease N^{pro} from Classical Swine Fever Virus (CSFV) used in this technology serves as an attractive tool for expression of therapeutic proteins in large amounts especially in E. coli. Medical applications require an authentic N-terminus of the recombinant proteins, which can be achieved by self-cleavage of N-terminally fused N^{pro} autoprotease. In addition, N^{pro} fusion technology also allows the expression of small or toxic peptides, which would be degraded immediately after their synthesis by host cell proteases (Achmüller et al., Nat. Methods 4 (2007), 1037-1043). As the expression of N^{pro} fusion proteins in E. coli leads to the formation of insoluble aggregates, known as inclusion bodies, appropriate resolving and renaturation protocols are required to obtain biological active proteins.

As already mentioned, in most cases solubilisation is carried out in chaotropic agents such as urea or guanidinium chloride at high concentrations in combination with reducing agents to abolish false formed disulfide bonds. Due to its simplicity refolding by dilution is widely used to initiate renaturation. Hence, large amounts of buffer are added to provide conditions, which allow the formation of the correct biological active structure. Whereas autoprotease cleavage and refolding of the released target molecule is usually performed in batch mode, the method according to the present invention enables the renaturation from solubilized inclusion bodies of N^{pro} autoprotease fusion proteins of therapeutic relevance using a new chromatography approach. According to the method of the present invention, the fusion protein can be bound to a multimodal resin column at a moderate conductivity range. During the elution into kosmotropic conditions the self-cleavage activity of N^{pro} is used to release the fusion partner with an authentic N-terminus. In comparison to the classical batch renaturation of N^{pro} fusions by rapid dilution in continuously stirred tank reactors with the technique according to the present invention, a significant increase in productivity can be achieved. The matrix assisted refold of the fusion proteins applying multimodal ligand chromatography material enables a significant lowering of the amount of buffer required after the solubilisation step and thus reduce costs. According to the present invention, renaturing of the fusion protein can be improved; the kosmotropic elution conditions support renaturing of the N^{pro} autoprotease. Renaturing of the N^{pro} autoprotease activate the autoproteolytic activity of the N^{pro} autoprotease and autoproteolytic cleavage of the fusion protein into N^{pro} autoprotease moiety and protein of interest can occur. According to the present invention, this renaturing and cleavage process is initiated already at the column before or during the elution from the multimodal resin. Although the cleavage process can be further conducted in batch mode afterwards (as usual), the early cleavage in the course of elution enables significant improvement of the overall process, especially an increase in the productivity of Npro cleavage. This allows also significant simplification and improvement if the process is conducted in large (industrial) scale. The specific binding of the fusion protein to the multimodal ligand material according to the present invention allows an elution of the protein by a very low amount of elution buffer, e.g. by about 2-fold, especially by a 2 to 3-fold column volume. This allows a lower volume for cleavage process and - at the same time - a higher fusion protein concentration in the renaturing set-up. The overall cleavage volume can therefore be significantly reduced, e.g. by at least 20 %, especially by at least 30 %, compared with the usual ion exchange chromatography material used in prior art methods.

The multimodal ligand material used in the process according to the present invention also allows a pre-purification of the fusion protein by the selective binding to the matrix. This also amounts to the reduction of cleavage time (which can be up to 30 % for the process according to the present invention, again, compared with previous set-ups).

Accordingly, in a preferred method the inclusion bodies were generated in a recombinant production system, preferably in a prokaryotic host cell, especially in E. coli host cells.

Preferred chaotropic conditions in step (b) correspond to a urea concentration of at least 3 M and/or not more than 8 M, preferably 3 M - 5 M. "Correspond to" means that either urea is present in the amount indicated or that another chaotropic substance (such as butanol, ethanol, guanidinium chloride, lithium perchlorate, magnesium chloride, phenol, propanol, sodium dodecyl sulfate, thiourea, etc.) is present in a concentration which leads to the same chaotropic effect (measured as increase of the entropy of the system.

The terms "kosmotrope" (order-maker) and "chaotrope" (disorder-maker) originally denoted solutes that stabilized, or destabilized respectively, proteins and membranes. Later they referred to the apparently correlating property of increasing, or decreasing respectively, the structuring of water. Such properties may vary dependent on the circumstances, method of determination or the solvation shell(s) investigated. An alternative term used for kosmotrope is "compensatory solute" as they have been found to compensate for the deleterious effects of high salt contents (which destroy the natural hydrogen bonded network of water) in osmotically stressed cells. Both the extent and strength of hydrogen bonding may be changed independently by the solute but either of these may be, and has been, used as measures of order making. It is, however, the effects on the extent of quality hydrogen bonding that is of overriding importance. The ordering effects of kosmotropes may be confused by their diffusional rotation, which creates more extensive disorganized junction zones of greater disorder with the surrounding bulk water than less hydrated chaotropes. Most kosmotropes do not cause a large scale net structuring in water.

Ionic kosmotropes (or: "antichaotropes" to distinguish them from non-ionic kosmotropes) should be treated differently from non-ionic kosmotropes, due mainly to the directed and polarized arrangements of the surrounding water molecules. Generally, ionic behaviour parallels the Hofmeister series. Large singly charged ions, with low charge density (e.g. SCN⁻, H₂PO₄⁻, HSO₄⁻, HCO₃⁻, I⁻, Cl⁻, NO₃⁻, NH₄⁺, Cs⁺, K⁺, (NH₂)₃C⁺ (guanidinium) and (CH₃)₄N⁺ (tetramethylammonium) ions; exhibiting weaker interactions with water than water with itself and thus interfering little in the hydrogen bonding of the surrounding water), are chaotropes whereas small or multiply-charged ions, with high charge density, are kosmotropes (e.g. SO₄²⁻, HPO₄²⁻, Mg²⁺, Ca²⁺, Li⁺, Na⁺, H⁺, OH⁻ and HPO₄²⁻, exhibiting stronger interactions with water molecules than water with itself and therefore capable of breaking water-water hydrogen bonds). The radii of singly charged chaotropic ions are greater than 1.06Å for cations and greater than 1.78Å for anions. Thus the hydrogen bonding between water molecules is more broken in the immediate vicinity of ionic kosmotropes than ionic chaotropes. Reinforcing this conclusion, a Raman spectroscopic study of the hydrogen-bonded structure of water around the halide ions F⁻, Cl⁻, Br⁻ and I⁻ indicates that the total extent of aqueous hydrogen bonding increases with increasing ionic size and an IR study in HDO:D₂O showed slow hydrogen bond reorientation around these halide ions getting slower with respect to increasing size. It is not unreasonable that a solute may strengthen some of the hydrogen bonds surrounding it (structure making; e.g. kosmotropic cations will strengthen the hydrogen bonds donated by the inner shell water molecules) whilst at the same time breaking some other hydrogen bonds (structure breaker; e.g. kosmotropic cations will weaken the hydrogen bonds accepted by the inner shell water molecules). Other factors being equal, water molecules are held more strongly by molecules with a net charge than by molecules with no net charge; as shown by the difference between zwitterionic and cationic amino acids.

Weakly hydrated ions (chaotropes, K⁺, Rb⁺, Cs⁺, Br⁻, I⁻, guanidinium⁺) may be "pushed" onto weakly hydrated surfaces by strong water-water interactions with the transition from strong ionic hydration to weak ionic hydration occurring where the strength of the ion-water hydration approximately equals the strength of water-water interactions in bulk solution (with Na⁺ being borderline on the strong side and Cl⁻ being borderline on the weak side). Neutron diffraction studies on two important chaotropes (guanidinium and thiocyanate ions) show their very poor hydration, supporting the suggestion that they preferentially interact with the protein rather than the water. In contract to the kosmotropes, there is little significant difference between the properties of ionic and nonionc chaotropes due to the low charge density of the former.

Optimum stabilization of biological macromolecule by salt requires a mixture of a kosmotropic anion with a chaotropic cation.

Chaotropes break down the hydrogen-bonded network of water, so allowing macromolecules more structural freedom and encouraging protein extension and denaturation. Kosmotropes are stabilizing solutes which increase the order of water (such as polyhydric alcohols, trehalose, trimethylamine N-oxide, glycine betaine, ectoine, proline and various other zwitterions) whereas chaotropes create weaker hydrogen bonding, decreasing the order of water, increasing its surface tension and destabilizing macromolecular structures (such as guanidinium chloride and urea at high concentrations). Recent work has shown that urea weakens both hydrogen bonding and hydrophobic interactions but glucose acts as a kosmotrope, enhancing these properties. Thus, when urea molecules are less than optimally hydrated (about 6 - 8 moles water per mole urea) urea hydrogen bonds to itself and the protein (significantly involving the peptide links) in the absence of sufficient water, so becoming more hydrophobic and hence more able to interact with further sites on the protein, leading to localized dehydration-led denaturation. Guanidinium is a planar ion that may form weak hydrogen bonds around its edge but may establish strongly-held hydrogen-bonded ion pairs to protein carboxylates, similar to commonly found quaternary structural arginine-carboxylate "salt" links. Also, guanidinium possesses rather hydrophobic surfaces that may interact with similar protein surfaces to enable protein denaturation. Both denaturants may cause protein swelling and destructuring by sliding between hydrophobic sites and consequently dragging in hydrogen-bound water to complete the denaturation.

Generally the kosmotropic/chaotropic nature of a solute is determined from the physical bulk properties of water, often at necessarily high concentration. The change in the degree of structuring may be found, for example, using NMR or vibrational spectroscopy. Protein-stabilizing solutes (kosmotropes) increase the extent of hydrogen bonding (reducing the proton and ¹⁷O spin-lattice relaxation times) whereas the NMR chemical shift may increase (showing weaker bonding e.g. the zwitterionic kosmotrope, trimethylamine N-oxide) or decrease (showing stronger bonding e.g. the polyhydroxy kosmotrope, trehalose). Trehalose shows both a reduction in chemical shift and relaxation time, as to a lesser extent does the protein stabilizer (NH₄)₂SO₄, whereas NaCl only shows a reduction in chemical shift and the protein destabilizer KSCN shows an increase in relaxation time and a reduction in chemical shift. Vibrational spectroscopy may make use of the near-IR wavelength near 5200 cm⁻¹ (v₂ + v₃ combination), which shifts towards longer wavelength (smaller wavenumber) when hydrogen bonds are stronger.

One of the most important kosmotropes is the non-reducing sugar α,α-trehalose. It should perhaps be noted that trehalose has a much more static structure than the reducing sugars, due to its lack of mutarotation, or the other common non-reducing disaccharide, sucrose, due to its lack of a furan ring.

Accordingly, the term "chaotropic conditions" has to be regarded individually on the nature of the liquid starting preparation (which may e.g. be a solution, a suspension, an emulsion, a two- or three phase liquid system, etc.), especially - in preparations containing more than one phase - on the aqueous phase of the preparation. Preferred chaotropic conditions according to the present invention are those which correspond to an urea concentration of 1 to 7 M, especially from 2 to 6 M (preferably in a buffered salt solution, such as 8.0g NaCl, 0.2g KCl, 1.44g Na₂HPO₄, 0.24g KH₂PO₄ ad 1000 ml with A. dest., pH 7.4 with HCl). As already mentioned above, correspondence of chaotropic conditions (as well as reduction of chaotropicity ("lower" or "less" chaotropic conditions")) may be easily determined by the methods mentioned above as well as by applying the teachings of the Hofmeister series. Addition of various substances in the starting liquid has to be checked in individual cases in order to provide optimum binding/non-aggregating conditions for binding. For example, the use of reduction agents should be optimised to correspond to an amount of 0.05 to 50 mM dithiothreitole (DTT), especially 0.1 to 10 mM DTT. Furthermore, also the addition of detergents may, as described above, influence the chaotropicity of the starting preparation. According to the present invention, binding of the fusion protein (= fusion polypeptide) is established under chaotropic, inactivating conditions. In order to induce refolding, conditions are changed to kosmotropic. In a preferred embodiment the step of refolding of the fusion protein is performed by the change from chaotropic to kosmotropic conditions via buffer exchange. Buffers can be alternatively gradually or instantaneously changed to kosmotropic conditions. In one preferred embodiment of the present invention the exchange of chaotropic buffer with kosmotropic buffer is conducted instantaneously, by application of the buffer as a plug. In another equally preferred embodiment of the present invention the exchange of buffers is conducted gradually.

Binding of the fusion protein to the column and/or refolding and cleaving of the fusion protein might be facilitated if the buffer exchange is accompanied by a temperature adjustment. This can, for example, be introduced by a cooling/heating jacket. Therefore, in a preferred embodiment, a cooling/heating jacket is applied for temperature adjustment; more preferably, the buffer is brought to the desired temperature prior to its application. In this way such temperature adjustment is achieved.

Upon change of conditions in the packed bed the fusion protein starts to refold and the part exerting the autoproteolytic function becomes active. As a result, the C-terminally fused polypeptide of interest is cleaved off at a distinct site defined by the specificity of the autoproteolytic part, thereby producing a homogeneous N-terminus of the protein of interest. Depending on the time required for refolding of the fusion protein, the velocity of the mobile phase with the kosmotropic buffer is reduced or stopped when all chaotropic buffer is displaced from the packed bed. After refolding is complete, the liberated protein of interest is washed out from the packed bed by further feeding of kosmotropic buffer. The N-terminal autoproteolytic part of the fusion protein as well as uncleaved fusion protein is eluted by conventional means, e. g. high salt concentration, a pH-change or NaOH, to regenerate the chromatography material. For regeneration the packed bed is washed with a buffer that strips the autoprotease from the adsorbent. These buffers comprise either acidic or alkaline solutions or organic solvents. After re-equilibration with starting buffer/chaotropic buffer the packed bed is ready for the next cycle.

For example, the fusion protein can be bound in presence of 3 to 5 M, especially 4 M urea. Urea is subsequently removed e.g. by detergent and lipid bilayer. When the binding of the fusion protein to the chromatography system according to the present invention has been accomplished, unbound contaminating components can easily be washed off the column. Such contaminating compounds might for example be host cell polypeptides and nucleic acids, which were occluded into or adsorbed on the inclusion bodies, and remain in the fusion protein solution/suspension after solubilisation, as well as residual components from an enzymatic cell disruption. After washing only the fusion protein remains bound to the column so that the following steps are conducted in a purified system.

Preferred kosmotropic conditions in step (d) correspond to a urea concentration of 0.1 to 1.5 M, preferably from 0.2 to 1 M, especially from 0.4 to 0.8 M.

Preferably, the elution buffer contains a buffer, especially a TRIS buffer or a phosphate buffer, Brij 58, a reducing agent, especially dithiothreitol (DTT) or dithioerythritol (DTE), an ion chelating agent, especially ethylenediaminetetraacetate (EDTA), a detergent, preferably a non-ionic detergent, especially polysorbate 20, 40, 60, or 80 or octyl phenol ethoxylate, a lauroyl amino acid, especially n-lauroyl-L-glutamate, an amino acid, especially L-arginine, L-histidine or L-lysine, a carbohydrate, especially sucrose, fructose or glucose, or mixtures thereof.

If L-arginine is used as an amino acid in the elution buffer, 100 mM to 1 M is a preferred concentration thereof in the buffer.

A specifically preferred embodiment of the present invention employs an elution buffer which comprises sucrose, preferably in a concentration of 100 to 1000 mM sucrose, especially of 250 to 750 mM.

According to a preferred embodiment, the elution buffer has a pH of 6 to 9, preferably 7 to 8.5, especially 7 to 8.5.

The present invention is based on the use of a specific binding material, the multimodal ligand resins. Such multimodal chromatographic material is known for several years and e.g. disclosed in WO 2004/024318 A1, WO 2004/078311 A1 or EP 2 017 875 A1. Multimodal ligands are able to interact with a target molecule in several different ways, e.g. coulombic attractions and mild hydrophobic interactions (WO 2004/024318 A1). A multimodal ligand is capable of providing at least two different, but co-operative, sites which interact with the substance to be bound. One of these sites gives hydrophobic interaction between the ligand and the fusion protein according to the present invention. The second site typically gives electron acceptor-donor interaction and/or hydrophobic and/or hydrophilic interactions and/or hydrogen bonding. Electron donor-acceptor interactions include interactions such as hydrogen-bonding, n-n, charge transfer, dipole-dipole, induced dipole etc. (WO 2004/078311 A1).

Preferred multimodal chromatographic material contains a ligand selected from a negatively charged 2- (benzoylamino) butanoic acid ligand, a phenylpropyl ligand, a positively charged N-Benzyl-N-methyl ethanolamine ligand, a N-hexyl ligand, a 4-Mercapto-Ethyl-Pyridine ligand, a 3-((3-methyl-5-((tetrahydrofuran-2-ylmethyl)-amino)-phenyl)-amino)-benzoic acid ligand or combinations thereof.

Preferably, the multimodal chromatography resin for use according to the present invention is selected from the following commercially available resins HEP Hypercel^{™}, PPA Hypercel^{™}; Capto Adhere^{™}, Capto MMC^{™}, or MEP Hypercel. For example, Capto MMC^{™} is a multimodal cation exchanger with high dynamic binding capacity at high conductivity, high volume throughput, new selectivity and smaller unit operations. The adsorption onto Capto MMC is salt tolerant, meaning that binding of proteins can be performed at the conductivity of the feed material. The medium is based on a highly rigid agarose base matrix that allows high flow rates and low back pressure at large scale. The material comprises a 2-(bezoylamino)butanoic acid residue and two 2-hydroxypropylether groups (linked via S). The Capto Adhere^{™} ligand is N-Benzyl-N-methyl ethanol amine.

According to a preferred embodiment, the protein of interest is a protein for therapeutic use in humans, preferably a human recombinant protein or a vaccination antigen.

Preferably, step (c) is performed at a pH which does not differ from the pI of the fusion protein by more than 1, especially not more than 0.5. The pH of the buffer is therefore preferably selected near the pI of the fusion protein.

Elution according to the present invention is preferably performed at higher ionic strength than binding and washing. High ionic strength is preferred for renaturing N^{pro} autoprotease and cleavage. For example step (d) is performed in the presence of a buffer comprising NaCl, preferably of 50 to 5000 mM NaCl, especially of 500 to 3000 mM NaCl.

Performing a washing step between steps (c) and (d) enables a higher purification of the fusion protein and further reduces the cleavage volume needed. In addition, it can further reduce cleavage time. Accordingly, a preferred embodiment of the method according to the present invention is **characterized in that** a washing step is performed between steps (c) and (d). Preferably, this washing step is performed at a pH being lower than the elution buffer, e.g. at a pH of between 5 and 9, especially between 5.5 and 7.5.

Although higher chaotropic conditions would support the solubilisation process, such conditions usually decrease the cleavage rate of the autoprotease. A cleavage rate which is too low does not allow a proper industrial use of the present method, at least on a large-scale set-up. However, the use of multimodal ligands according to the present invention allows a proper resolubilisation process and a proper cleavage at low cleavage volume and high cleavage rates.

Preferred examples for the N^{pro} autoprotease moiety of the fusion protein are naturally occurring versions of the N^{pro} autoprotease or, preferably, deletion mutants of naturally occurring versions of the N^{pro} autoprotease. Such deletions, of course, must not lead to inactivation of proteolytic activity. For example, amino acids 1 to 21 (the amino acid numbering follows the numbering of most naturally occurring N^{pro} autoprotease sequences of CSFV, such as listed in Becher et al., J. Gen. Virol. 78 (1997), 1357-1366) can be deleted without affecting proteolytic activity. It is therefore preferred to use an N^{pro} autoprotease lacking amino acids 1 to 21. These preferred autoproteases with proteolytic activity therefore start with the GluPro motif (at positions 22/23), preferably followed by a (Val/Leu) (Tyr/Phe) motif (amino acids 24 and 25 of N^{pro}). Another example for possible deletion without affecting proteolytic activity is amino acids 148 to 150 (e.g. ThrProArg in "EDDIE" (Achmüller et al., 2007) or GluProArg in the Alfort sequence (Becher et al., 1997)).

Sequence variations occur between the various natural isolates (see e.g. GenBank or EMBL databases); also selected mutations have been provided with improved properties (see WO 2006/113957 A; Achmüller et al., 2007; Achmüller, PhD thesis, March 2006, University of Innsbruck (AT)): For example,
- Cys112, Cys134 and Cys138 can be replaced by another amino acid residue, preferably Glu;
- His5, Lys16, Asn35, Arg53, Gly54, Arg57, Leu143, Lys145 and/or Arg150 can be replaced by another amino acid residue, preferably arginine (R) 53 with glutamic acid (E), glycine (G) 54 with aspartic acid (D), arginine (R) 57 with glutamic acid (E), and/or leucine (L) 143 with glutamine (Q);
- Val24, Ala27, Leu32, Gly54, Leu75, Ala109, Val114, Val121, Leu143, Ile155 and/or Phe158 can be replaced by another amino acid residue, preferably threonine or serine, especially alanine (A) 109, valine (V) 114, isoleucine (I) 155 and/or phenylalanine (F)158;
- Ala28, Ser71 and/or Arg150 can be replaced by another amino acid residue, preferably glutamic acid (E), phenylalanine (F) and/or with histidine (H), especially alanine (A) 28 can be replaced with glutamic acid (E), serine (S) 71 can be replaced with phenylalanine (F) and arginine (R) 150 can be replaced with histidine (H).

Preferred autoproteases can be chosen also according to the fusion partner ("protein of interest"). For example, preferred sequences are the N^{pro} sequences disclosed in WO 2006/113957 A (as SEQ.ID.NOs. 1-5, 32/33, 92-98, especially SEQ.ID.NO 5 ("EDDIE")).

The present method can in principle be applied for production of any protein of interest, especially for all proteins known to be producible by the N^{pro} autoprotease technique. A "protein of interest" may therefore be any protein which does - on a gene level - not naturally occur in direct 5'-3' connection with an N^{pro} autoprotease. Since the method according to the present invention is suitable for large-scale manufacturing and pharmaceutical good manufacturing practice, it is preferred to produce a protein for therapeutic use in humans with the present method, preferably a human recombinant protein or a vaccination antigen.

The process parameters can be optimised for each set-up, preferably depending on the N^{pro} autoprotease used and on the protein of interest to be produced.

The present invention is carried out with the N^{pro} technology. This technology is disclosed e.g. in WO 01/11057 A, WO 01/11056 A, WO 2006/113957 A, WO 2006/113958 A, WO 2006/113959 A, and Achmüller et al., Nat. Meth. 4 (2007), 1037-1043. In general terms, the N^{pro} technology relates to a process for the recombinant production of a heterologous protein of interest, comprising (i) cultivation of a bacterial host cell which is transformed with an expression vector which comprises a nucleic acid molecule which codes for a fusion protein, the fusion protein comprising a first polypeptide which exhibits the autoproteolytic function of an autoprotease N^{pro} of a pestivirus, and a second polypeptide which is connected to the first polypeptide at the C-terminus of the first polypeptide in a manner such that the second polypeptide is capable of being cleaved from the fusion protein by the autoproteolytic activity of the first polypeptide, and the second polypeptide being a heterologous protein of interest, wherein cultivation occurs under conditions which cause expression of the fusion protein and formation of corresponding cytoplasmic inclusion bodies, (ii) isolation of the inclusion bodies from the host cell, (iii) solubilisation of the isolated inclusion bodies, (iv) dilution of the solubilisate to give a reaction solution in which the autoproteolytic cleavage of the heterologous protein of interest from the fusion protein is performed, and (v) isolation of the cleaved heterologous protein of interest.

This technology is suited for a large variety of proteins of interest. For the purpose of the present invention, the terms "heterologous protein", "target protein", "polypeptide of interest" or "protein of interest" (and the like) mean a polypeptide which is not naturally cleaved by an autoprotease N^{pro} of a Pestivirus from a naturally occurring fusion protein or polyprotein (i.e. a polypeptide being different than the naturally following amino acids 169ff of the Pestivirus polyprotein encoding the structural Protein C and subsequent viral proteins). Examples of such heterologous proteins of interest are industrial enzymes (process enzymes) or polypeptides with pharmaceutical, in particular human pharmaceutical, activity.

Due to its autocatalytic cleavage it enables synthesis of proteins with an authentic N-terminus which is especially important for pharmaceutical applications. Furthermore, not only large proteins ("proteins of interest") but also small peptides can be stably expressed by C-terminal linking to N^{pro}. A high expression rate forces the fusion protein into inclusion bodies. After purification, N^{pro} is refolded and cleaves itself off.

It is essential that the protein of interest to be produced by the present invention is attached C-terminally after Cys168 of the N^{pro} autoprotease, because this is the cleavage site where the peptidic bond between the C-terminus of the N^{pro} moiety (at Cys168) and the protein of interest is cleaved in step (c) according to the present invention.

Examples of preferred proteins of interest with human pharmaceutical activity are cytokines such as interleukins, for example IL-6, interferons such as leukocyte interferons, for example interferon a2B, growth factors, in particular haemopoietic or wound-healing growth factors, such as G-CSF, erythropoietin, or IGF, hormones such as human growth hormone (hGH), antibodies or vaccines. Also very short polypeptides having only 5 to 30 amino acid residues can be produced as protein of interest by the present technology. The N^{pro} technology has specific advantages in an expression system making use of inclusion bodies, because the strong aggregation bias of the fused autoprotease facilitates the formation of inert inclusion bodies, almost independent of the fusion partner. Accordingly, almost any protein of interest is producible with the present system in high amounts and yields. Reports are e.g. available for expression of synthetic interferon-a1; toxic gyrase inhibitor CcdB, a short 16-residue model peptide termed pep6His (SVDKLAAALEHHHHHH), human proinsulin, synthetic double domain D of staphylococcal protein A (sSpA-D₂), keratin-associated protein 10-4 (KRTAP10-4), synthetic green fluorescent protein variant (sGFPmut3.1), synthetic inhibitorial peptide of senescence evasion factor with N-terminal cysteine (C-sSNEVi), synthetic inhibitorial peptide of senescence evasion factor with randomized amino acid sequence with C-terminal cysteine (sSNEVscr-C); recombinant human monocyte chemoattractant protein 1 (rhMCP-1). So far, the only limitations with respect to high yields have been suspected for chaperones and proteins with comparable properties of supporting protein folding. Such proteins as fusion partners could suppress the aggregation bias of an N^{pro} molecule, leading to lower yields due to less aggregation. Nevertheless, the present technology can even be applied for expressing such proteins counter-acting aggregation.

The fusion protein according to the present invention can additionally contain auxiliary sequences, such as affinity tags or refolding aid moieties; it may also contain more than one protein of interest (it can e.g. contain two or three or four or even more proteins of interest which may be separated from each other at a later stage or even at the same stage as the cleavage by the Npro autoprotease).

The present invention also relates to an expression vector encoding for a fusion protein comprising an N^{pro} autoprotease and the protein of interest. In the expression vector to be employed in the process according to the present invention, the fusion polypeptide is operably linked to at least one expression control sequence. Expression control sequences are, in particular, promoters (such as the lac, tac, T3, T7, trp, gac, vhb, lambda pL or phoA promoter), ribosome binding sites (for example natural ribosome binding sites which belong to the abovementioned promoters, cro or synthetic ribosome binding sites), or transcription terminators (for example rrnB T1T2 or bla).

The vector may also contain sequences encoding fusion domains, as described below, that are present at the N-terminal end of the fusion polypeptide (= fusion protein) and that are required for its binding to the affinity chromatography system, e.g. polyamino acids like polylysine or, for immunoaffinity chromatogography, so-called "epitope tags", which are usually short peptide sequences for which a specific antibody is available. Well known epitope tags for which specific monoclonal antibodies are readily available include FLAG, influenza virus haemagglutinin (HA), and c-myc tags.

In a preferred embodiment of the present invention, the expression vector is a plasmid.

Another aspect of the present invention relates to a host cell, preferably a prokaryotic host cell, especially an E. coli host cell, containing an expression vector according to the present invention. The transformed bacterial host cell, i.e. the expression strain, is cultivated in accordance with microbiological practice known per se. The host strain is generally brought up starting from a single colony on a nutrient medium, but it is also possible to employ cryo-preserved cell suspensions (cell banks). The strain is generally cultivated in a multistage process in order to obtain sufficient biomass for further use.

On a small scale, this can take place in shaken flasks, it being possible in most cases to employ a complex medium (for example LB broth). However, it is also possible to use defined media (for example citrate medium). Since in the preferred embodiment of the present invention it is intended that the expressed fusion polypeptide is in the form of insoluble inclusion bodies, the culture will in these cases be carried out at relatively high temperature (for example 30°C or 37°C). Inducible systems are particularly suitable for producing inclusion bodies (for example with the trp, lac, tac or phoA promoter).

On a larger scale, the multistage system consists of a plurality of bioreactors (fermenters), it being preferred to employ defined nutrient media. In addition, it is possible greatly to increase biomass and product formation by metering in particular nutrients (fed batch). Otherwise, the process is analogous to the shaken flask. In the process according to the present invention, the inclusion bodies are isolated from the host cell in a manner known per se. For example, after the fermentation has taken place, the host cells are harvested by centrifugation, micro filtration, flocculation or a combination thereof, preferably by centrifugation. The wet cell mass is disintegrated by mechanical, chemical or physical means such as high pressure homogenizer, beads mills, French press, Hughes press, osmotic shock, detergents, enzymatic lysis or a combination thereof. Preferably, disruption of the cells takes place by high pressure homogenization. In the preferred embodiment where the recombinant fusion polypeptide is deposited as inclusion bodies, the inclusion bodies can be obtained for example by means of high-pressure dispersion or, preferably, by a simple centrifugation at low rotor speed. The inclusion bodies are separated by centrifugation or microfiltration or a combination thereof. The purity in relation to the desired polypeptide of interest can then be improved by multiple resuspension of the inclusion bodies in various buffers, for example in the presence of NaCl (for example 0.5 1.0 M) and/or detergent (for example Triton X 100). Preferably the purity of the inclusion body preparation is improved by several washing steps with various buffers (e.g. 0.5 % Deoxycholate followed by two times 1 M NaCl solution and finally distilled water). This usually results in removal of most of the foreign polypeptides from the inclusion bodies.

The present invention is further described by the following examples and the drawing figures, yet without being restricted thereto.
Figure 1: The process according to the Npro technology.
Figure 2: The buffer dependent cleavage over time (Buffer 1: Tris, L-arginine, EDTA, non-ionic detergent, reducing agent, urea, NaCl, pH 8; Buffer 2: Tris, reducing agent, EDTA, Glycerol, urea, pH 7.5; Buffer 3: 1.5 M Tris, 0.25 M sucrose, 2 mM EDTA, 20 mM DTT, 0.6 M urea, pH 7.5; Buffer 4: Tris, L-arginine, sucrose, EDTA, non-ionic detergent, DTT, urea, NaCl, pH 8).
Figure 3: Dynamic and static binding capacity.
Figure 4: Purification effect of matrix assisted refolding (MAR); M: SeeBlue Marker; MAR: Matrix assisted refold (eluate); B: Batch refold.
Figure 5: Comparison of MAR vs. batch cleavage.
Figure 6: Determination of the dynamic binding capacity at 10 % Break Through.

### Examples

### Materials and methods

### Protein expression

Autoprotease N^{pro} was cloned into vectors harboring as protein of interest. The vectors were transformed into E.coli by electroporation and cells were grown over night at 37°C. Cells were diluted 1:100 and incubated at 37°C until OD₆₀₀ reached 0.5. Protein expression was induced by addition of 1M IPTG (isopropyl β-D-1-thiogalactopyranoside) to a final concentration of 1 mM IPTG followed by an incubation for four hours at 37°C. Cells were harvested by centrifugation. Lysis was carried out using a french press. Inclusion bodies were harvested by a further centrifugation step.

Dynamic and static binding capacity

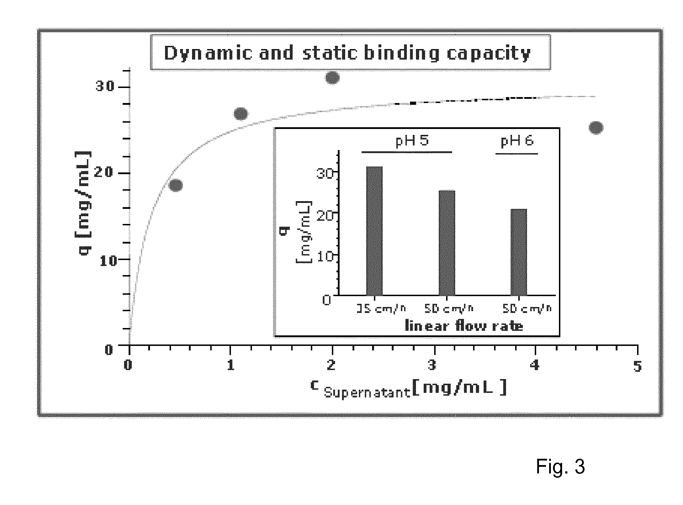

Fig.3. The buffer dependent cleavage over time

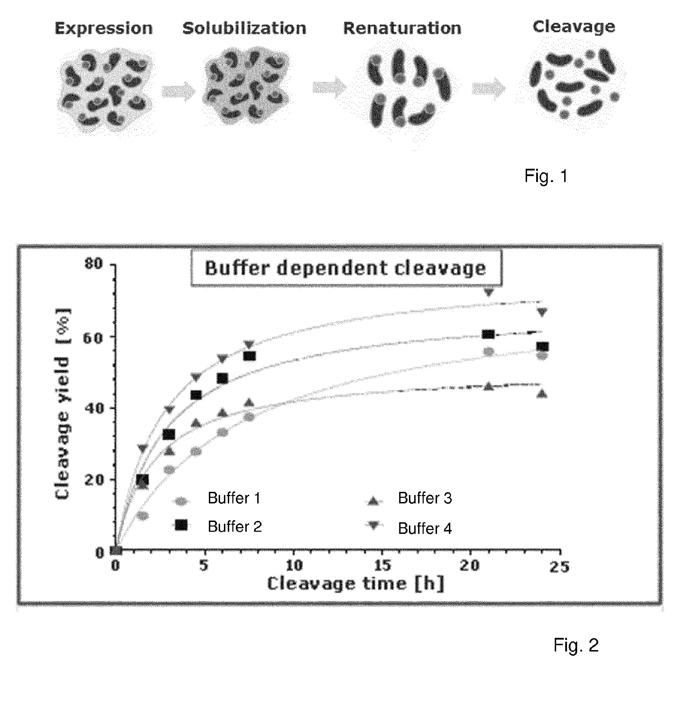

Fig. 2: Buffer 1: Tris, L-arginine, EDTA, non-ionic detergent, reducing agent, urea, NaCl, pH 8; Buffer 2: Tris, reducing agent, EDTA, Glycerol, urea, pH 7.5; Buffer 3: 1.5 M Tris, 0.25 M sucrose, 2 mM EDTA, 20 mM DTT, 0.6 M urea, pH 7.5; Buffer 4: Tris, L-arginine, sucrose, EDTA, non-ionic detergent, DTT, urea, NaCl, pH 8.

Purification with matrix assisted refolding (MAR)

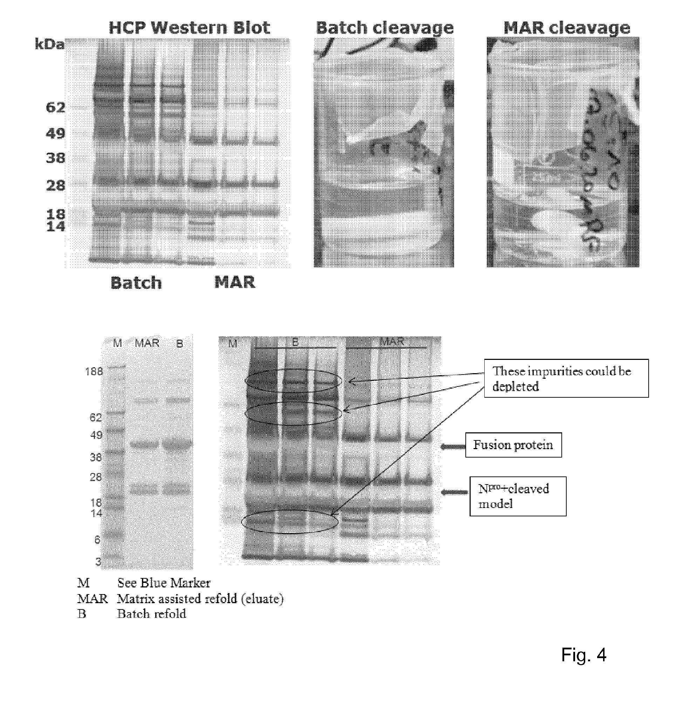

Fig.4. Through the binding of the Npro Fusion protein to the chromatographic resin a selective capture could be established. For comparison a HCP Western Blot for residual HCPs of E. coli of the MAR eluate and the batch refolds was performed (Fig. 4).

### Batch cleavage

Figs. 4 and 5.

Determination of the dynamic binding capacity at 10 % Break Through

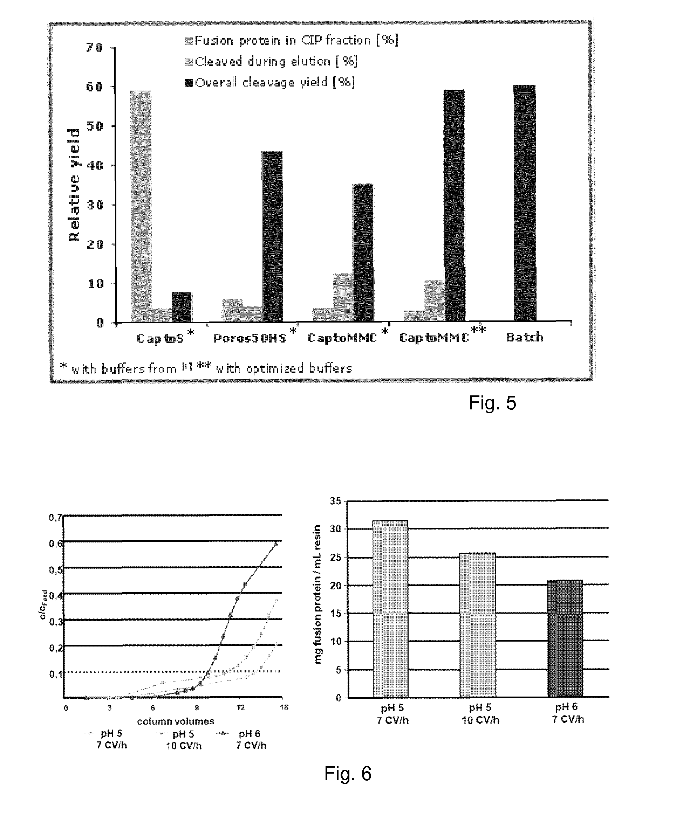

For the determination of the dynamic binding capacity a CaptoMMC column with the dimensions 10 x 48 mm was used. The solubilization of the inclusion bodies of the model protein was performed like described before. After 1+2 dilution of the solubilized inclusion bodies with equilibration buffer the column was loaded with the given flow rate. The flow through of the column was collected in 0.5 mL fractions and the fusion protein concentration of each fraction was determined by reversed phase HPLC. The results were plotted against the normalized feed flow volume and the resulted breakthrough with the given parameters was calculated (Fig. 6).

For the calculation of the productivity of the both processes - MAR and Batch - of an Npro fusion protein the following calculation was used:
- Refold of the fusion protein of an 1000 L batch refold Batch-Refold:
- 1000 L with 4 mg/mL fusion protein resulting in 4000 g fusion protein
- 50.8% of fusion protein represents the model protein -> 2032 g model protein
- A yield of 70 % cleavage results in 1422.2 g model protein within 24 h
- Productivity = 1422.2 g model/(1000 L buffer *24 h) = 0.059 g model protein / L*h

MAR:
- Load ratio: 17.7 mg/mL
- Column volume to bind 4000 g fusion protein would be 225.98 L
- An average elution volume of 2.55 column volumes results in 576.27 L eluate
- With an overall yield after 24 h of 59 % 1198.9 g model protein were produced
- For the productivity calculation 1 hour of operation of the MAR technique must be added
- Productivity = 1198.9 g model/(576.27 L buffer *25 h) = 0.0832 g model protein / L*h

This results in a 41% increased productivity with the new method

### Results

As can be seen from Figs. 2 to 6, the method according to the present invention results in a higher concentration of the protein of interest in the elution buffer and a significant shortening of process time. Further, a higher purity of the final product can be obtained by the present process which also requires less cleavage buffer. Overall productivity is therefore significantly increased by the present invention.

## Claims

1. Method for producing a recombinant protein of interest, **characterised in** by the following steps:
(a) providing a fusion protein comprising an N^{pro} autoprotease moiety and a protein of interest moiety in inclusion bodies,
(b) solubilising the fusion protein in the inclusion bodies by subjecting the inclusion bodies to chaotropic conditions,
(c) binding the fusion protein of the solubilised inclusion bodies to a multimodal chromatographic material under chaotropic conditions,
(d) eluting the fusion protein from the multimodal chromatographic material with an elution buffer and allowing the fusion protein to be cleaved by the N^{pro} autoprotease moiety under kosmotropic conditions, wherein the recombinant protein of interest is cleaved from the fusion protein, and
(e) recovering the protein of interest.

2. Method according to claim 1, **characterized in that** the inclusion bodies were generated in a recombinant production system, preferably in a prokaryotic host cell, especially in E. coli host cells.

3. Method according to claim 1 or 2, **characterized in that** the chaotropic conditions in step (b) correspond to a urea concentration of at least 3 M and/or not more than 8 M, preferably in the range of 3 M to 5 M.

4. Method according to any one of claims 1 to 3, **characterized in that** the kosmotropic conditions in step (d) correspond to a urea concentration of 0.1 to 1.5 M, preferably from 0.2 to 1 M, especially from 0.4 to 0.8 M.

5. Method according to any one of claims 1 to 4, **characterized in that** the elution buffer contains a buffer, especially a TRIS buffer or a phosphate buffer, a reducing agent, especially dithiothreitol (DTT) or dithioerythritol (DTE), an ion chelating agent, especially ethylenediaminetetraacetate (EDTA), a detergent, preferably a non-ionic detergent, especially polysorbate 20, 40, 60, or 80 or octyl phenol ethoxylate, Brij 58, a lauroyl amino acid, especially lauroyl-L-glutamate, an amino acid, especially L-arginine, L-histidine or L-lysine, a carbohydrate, especially sucrose, fructose or glucose, or mixtures thereof.

6. Method according to any one of claims 1 to 5, **characterized in that** the elution buffer has a pH of 6 to 9, preferably 7 to 8.5, especially 7 to 8.5.

7. Method according to any one of claims 1 to 6, **characterized in that** the multimodal chromatographic material contains a ligand selected from a negatively charged 2- (benzoylamino) butanoic acid ligand, a phenylpropyl ligand, a positively charged N-Benzyl-N-methyl ethanolamine ligand, a N-hexyl ligand, a 4-Mercapto-Ethyl-Pyridine ligand, a 3-((3-methyl-5-((tetrahydrofuran-2-ylmethyl)-amino)-phenyl)-amino)-benzoic acid ligand or combinations thereof.

8. Method according to any one of claims 1 to 7, **characterized in that** the protein of interest is a protein for therapeutic use in humans, preferably a human recombinant protein or a vaccination antigen.

9. Method according to any one of claims 1 to 8, **characterized in that** step (c) is performed at a pH which does not differ from the pI of the fusion protein by more than 1, especially not more than 0.5.

10. Method according to any one of claims 1 to 9, **characterized in that** step (d) is performed in the presence of a buffer comprising NaCl, preferably of 50 to 5000 mM NaCl, especially of 500 to 3000 mM NaCl.

11. Method according to any one of claims 1 to 10, **characterized in that** a washing step is performed between steps (c) and (d).
